# FASCICULE DE BREVET EUROPEEN

(11) **EP 4 031 525 B1**
(45) Date de publication et mention de la délivrance du brevet: **20.03.2024**
(21) Numéro de dépôt: 20804613.6
(22) Date de dépôt: 20.10.2020
(51) Int. Cl.: C07C 249/02, D21H 21/14, D21H 21/24, D21H 21/28

(54) **PROCÉDÉ DE PRÉPARATION D'UN COMPOSÉ COMPRENANT AU MOINS UNE FONCTION IMINE PAR UNE RÉACTION DE CONDENSATION SPÉCIFIQUE ET APPLICATION PARTICULIÈRE DE CE PROCÉDÉ AU DOMAINE DE LA COLORATION**
VERFAHREN ZUR HERSTELLUNG EINER VERBINDUNG MIT MINDESTENS EINER IMINFUNKTION DURCH EINE SPEZIFISCHE KONDENSATION UND SPEZIELLE ANWENDUNG DIESESES VERFAHRENS AUF DEM GEBIET DER FARBE
PROCESS FOR THE PREPARATION OF A COMPOUND COMPRISING AT LEAST ONE IMINE FUNCTION BY A SPECIFIC CONDENSATION REACTION AND PARTICULAR APPLICATION OF THIS PROCESS TO THE FIELD OF COLOURING

(30) Priorité: 29.10.2019 FR 1912128
(43) Date de publication de la demande: 27.07.2022
(73) Titulaire: Commissariat à l'Energie Atomique et aux Energies Alternatives, 75015 Paris (FR)
(72) Inventeur: PONCELET, Olivier, 38054 GRENOBLE Cedex 09 (FR); AUGER, Aurélien, 38054 GRENOBLE Cedex 09 (FR)
(74) Mandataire: Brevalex
(86) Numéro de dépôt international: PCT/FR2020/051882
(87) Numéro de publication internationale: WO 2021/084185

(56) Documents cités:
- WO-A1-2004/046087
- WO-A1-2017/158302
- WO-A2-2008/057312

## Description

### DOMAINE TECHNIQUE

La présente invention a trait à un procédé de préparation d'un composé comprenant au moins une fonction imine par une réaction de condensation spécifique, ce procédé pouvant trouver application dans tous les domaines nécessitant la transformation des propriétés d'un composé de base par l'introduction d'un groupe fonctionnel sur celui-ci *via* la formation d'au moins une fonction imine, et en particulier dans celui de la coloration.

Ce domaine d'application est particulièrement important face à la nécessité de pouvoir disposer de produits colorés, tels que des papiers, présentant une bonne tenue de la coloration à la lumière et une bonne rétention de celle-ci même après exposition à l'humidité ou contact simple ou prolongé avec différents types de solvants, notamment lorsque le produit coloré est destiné à constituer un produit de packaging.

De manière traditionnelle, la fabrication de papiers colorés comprend les étapes suivantes :
- la préparation d'une suspension aqueuse comprenant des fibres cellulosiques et au moins un colorant soluble dans l'eau présentant une affinité chimique pour les fibres et éventuellement un ou plusieurs fixateurs, de sorte à améliorer la fixation du ou des colorants sur les fibres ;
- la formation des feuilles en déposant la suspension aqueuse sur une toile permettant l'égouttage de la suspension.

Ce mode de fabrication présente toutefois l'inconvénient de générer des effluents aqueux comprenant du colorant et, par conséquent, une gestion de ces effluents. Qui plus est, il ne permet pas d'éviter le phénomène de relargage des colorants incorporés dans le papier, en particulier, après une exposition prolongée avec un milieu chargé en humidité, ce d'autant plus que les colorants ne sont pas liés aux fibres par des liaisons chimiques fortes, la fixation se faisant plutôt par simple adsorption chimique et qu'une partie des colorants reste simplement piégée dans le réseau fibreux.

Pour contourner l'inconvénient lié à la gestion des effluents aqueux pollués, des papiers colorés ont été produits, de manière alternative, en déposant le ou les colorants, par exemple, au moyen d'une presse encolleuse, sur les feuilles de papier préalablement formées, l'inconvénient découlant de cette technique étant la difficulté de répartir uniformément le ou les colorants à la surface des feuilles. Qui plus est, le ou les colorants sont présents uniquement en surface et non dans le coeur des feuilles, ce qui contribue à une coloration peu stable et susceptible d'évoluer substantiellement lors du vieillissement des feuilles.

Pour obtenir une coloration plus profonde et plus stable, les efforts de recherche se sont axé sur la mise au point de techniques innovantes d'imprégnation des colorants avec le papier en misant notamment sur des vecteurs d'imprégnation performants, tels que l'utilisation d'un milieu supercritique pour véhiculer le ou les colorants au coeur des fibres constitutives du papier.

Ainsi, dans FR 3 015 988, il est question d'un procédé d'imprégnation d'un papier par un agent d'imprégnation, tel qu'un colorant, comprenant les étapes suivantes :
- une étape préalable d'une formation des feuilles de papier, lesquelles incorporent un additif spécifique apte à recevoir et piéger l'agent d'imprégnation, cet additif pouvant être un polymère stimulable, tel que du PEOX ou du PEG ; des particules organiques ou inorganiques de type hydrophiles et comportant des cavités hydrophobes, telles que des cyclodextrines ou des argiles modifiées ou des molécules amphiphiles ;
- une étape d'imprégnation des feuilles de papier avec l'agent d'imprégnation par mise en contact de celles-ci avec ledit agent au moyen d'un fluide supercritique, tel que du CO₂ supercritique.

Si ce procédé permet une imprégnation jusqu'au coeur des feuilles par le colorant et une coloration satisfaisante, la fixation du colorant *via* l'additif est une fixation non covalente donc sujette à une possibilité de relargage du colorant en milieu humide et les papiers résultant de cette imprégnation présentent une tenue à la lumière insatisfaisante.

Dans WO 2015/140750, le procédé de coloration de papier décrit met également en oeuvre un milieu supercritique et plus spécifiquement, comprend les étapes suivantes :
- une étape de mise en contact de fibres cellulosiques avec un composé dérivé de l'urée de formule R-NH-CO-NH₂ avec R représentant une chaîne hydrocarbonée linéaire ou ramifiée, saturée ou insaturée, et comprenant au moins 3 atomes de carbone ou représentant un radical hydrocarboné cyclique, saturé ou insaturé, lié directement ou *via* un groupe méthylène ou éthylène à l'atome d'azote, moyennant quoi il se forme une liaison carbamate entre ce composé et les fibres cellulosiques ;
- une étape de mise en contact avec les fibres cellulosiques ainsi modifiées avec au moins un colorant hydrophobe, en milieu CO₂ supercritique.

Dans ce procédé, la liaison entre le colorant et les fibres s'effectue par un phénomène d'adsorption du colorant au niveau des motifs hydrocarbonés, c'est-à-dire des chaînes hydrocarbonées ou des radicaux hydrocarbonés cycliques du carbamate de cellulose. Cependant, ce type de liaison peut s'avérer insuffisante pour garantir une tenue de la coloration dans le temps et empêcher le relargage des colorants, notamment lorsque les papiers ainsi colorés sont soumis à un contact prolongé à un milieu humide ou comprenant des vapeurs de solvant organique.

Enfin, dans WO2017/158302, il est également question d'un procédé d'imprégnation d'un papier par une molécule d'intérêt, telle qu'un colorant, en milieu supercritique, l'imprégnation se produisant grâce à l'adjonction au papier à coeur et/ou en surface d'un additif polymérique introduit sous forme d'un latex (par exemple, une dispersion ionique aqueuse de particules de polymère, dont les chaînes comportent des groupements basiques au sens de Lewis, tels que des groupements éthers, carbonyles, carboxyles ou phényles), cet additif polymérique permettant le piégeage de la molécule d'intérêt. Toutefois, ce procédé, même s'il permet l'obtention d'une coloration efficace, nécessiterait des améliorations pour augmenter la résistance de la coloration à un lessivage du papier coloré après mise en contact de celui-ci avec un solvant organique, tel que l'acétone ou l'éthanol ou pour améliorer la tenue de la coloration à la lumière.

Au vu de ce qui existe et en vue de surmonter les inconvénients des procédés mentionnés ci-dessus, les auteurs de la présente invention se sont fixé pour objectif, tout en profitant des avantages inhérents à l'utilisation d'un milieu supercritique, de mettre au point un procédé spécifique de fixation d'un colorant sur un composé cellulosique, tel que le papier, la fixation s'établissant de manière covalente entre ledit colorant et ledit composé *via* la formation d'un groupe de liaison spécifique en milieu supercritique.

Sur la base de cet objectif, les auteurs de la présente invention ont découvert, de manière surprenante, qu'il est possible, en milieu supercritique, de faire réagir deux composés comportant respectivement un groupe carbonyle et un groupe amine pour former entre eux un groupe de liaison imine, ouvrant ainsi le champ de l'invention à tous les domaines nécessitant la transformation des propriétés d'un composé de base par l'introduction d'un groupe fonctionnel sur celui-ci *via* la formation d'au moins d'un groupe de liaison imine.

### EXPOSÉ DE L'INVENTION

Ainsi, l'invention a trait à un procédé de fabrication d'un composé comprenant au moins un groupe imine, ledit procédé comprenant une étape de réaction entre un premier composé comprenant au moins un groupe amine et un second composé comprenant au moins un groupe carbonyle, ladite étape de réaction étant réalisée en présence d'au moins un fluide supercritique.

Grâce à l'utilisation d'au moins un fluide supercritique pour mettre en oeuvre cette réaction, il a été constaté une vitesse de réaction beaucoup plus rapide par rapport à une réaction similaire conduite dans un milieu comprenant un solvant organique dans des conditions non supercritiques (par exemple, de 30 minutes à 3 heures avec le fluide supercritique contre plusieurs jours, voire 1 semaine avec le milieu en solvant classique).

Par fluide supercritique, il s'entend un fluide porté à une pression et une température au-delà de son point critique, correspondant au couple de température et de pression (respectivement Tc et Pc), pour lequel la phase liquide et la phase gazeuse présentent la même densité et au-delà duquel le fluide se situe dans son domaine supercritique. Dans des conditions supercritiques, le fluide présente un pouvoir de dissolution très accrue par rapport au même fluide dans des conditions non supercritiques et facilite de ce fait la solubilisation du premier composé et/ou du second composé facilitant ainsi la réactivité entre eux.

De préférence, l'étape de réaction est réalisée en présence d'un seul fluide supercritique, qui est, de préférence, du CO₂ supercritique, notamment en raison de sa température critique faible (31°C), ce qui permet de mettre en oeuvre la réaction à basse température sans risque de dégradation du premier composé et/ou du second composé. Plus précisément, le CO₂ supercritique s'obtient en chauffant du dioxyde de carbone au-delà de sa température critique (31°C) et en le comprimant au-dessus de sa pression critique (73 bars). Qui plus est, le CO₂ supercritique est non inflammable, non toxique, relativement bon marché et ne nécessite pas de retraitement à l'issue du procédé, comparativement à des procédés impliquant l'utilisation de solvant organique.

Comme mentionné ci-dessus, le procédé de l'invention comprend une étape de réaction entre un premier composé comprenant au moins un groupe amine et un second composé comprenant au moins un groupe carbonyle, ladite étape de réaction étant réalisée en présence d'au moins un fluide supercritique (étant entendu que le ou les groupes amines du premier composé et le ou les groupes carbonyles du second composé réagissent ensemble pour former le ou les groupes imines, le composé issu de l'étape de réaction résultant ainsi d'une réaction de condensation entre le premier composé et le second composé).

Concernant le premier composé, le ou les groupes amines peuvent être des groupes amines primaires (c'est-à-dire des groupes de formule -NH₂) et concernant le second composé, le ou les groupes carbonyles peuvent être des groupes aldéhydes, un groupe amine primaire et un groupe aldéhyde étant à même de réagir efficacement par une réaction de condensation pour former un groupe imine.
Il s'entend, par groupe imine, un groupe répondant à la formule suivante : 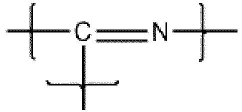 les deux accolades placées au niveau de l'atome de carbone indiquant que celui-ci est lié à deux autres atomes pour assurer sa tétravalence, ces deux autres atomes appartenant à un reste du second composé et l'accolade placée au niveau de l'atome d'azote indiquant que celui-ci est lié à un autre atome pour assurer sa trivalence, l'autre atome appartenant à un reste du premier composé.

Concernant le premier composé et le second composé, il s'entend que l'un au moins desdits composés est apte à se solubiliser dans le fluide supercritique utilisé dans le cadre du procédé de l'invention.

L'étape de réaction peut être mise en oeuvre dans un réacteur, par exemple, un autoclave, par la succession d'opérations suivantes :
- une opération de mise en contact du premier composé, du second composé et du fluide dans des conditions non supercritiques ;
- une opération de mise en conditions supercritiques du mélange comprenant le fluide, le premier composé et le second composé, les conditions supercritiques une fois établies étant maintenues jusqu'à achèvement de l'étape de réaction.

L'étape de réaction peut aussi être mise en oeuvre dans un réacteur, par exemple, un autoclave, par la succession d'opérations suivantes :
- une opération d'introduction dans le réacteur d'un fluide dans des conditions non supercritiques ;
- une opération de mise en conditions supercritiques du fluide ;
- une opération d'introduction dans le réacteur du premier composé et du second composé, les conditions supercritiques étant maintenues jusqu'à achèvement de l'étape de réaction.

Le procédé de l'invention peut consister, en particulier, en un procédé de modification des propriétés d'un composé de base (le premier composé ou le second composé) par l'introduction d'un groupe fonctionnel (appartenant au premier composé lorsque le composé de base est le second composé ou appartenant au second composé lorsque le composé de base est le premier composé) sur celui-ci via la formation d'au moins un groupe de liaison imine par réaction entre le ou les groupes amines du premier composé et le ou les groupes carbonyles du second composé, la modification des propriétés pouvant consister en une coloration.

Plus spécifiquement, le procédé de l'invention peut être un procédé de coloration, auquel cas l'un des composés, à savoir le premier composé ou le second composé, peut être un composé colorant (plus spécifiquement, un composé comprenant au moins un groupe chromophore) et l'autre composé (à savoir, le premier composé si le second composé est un composé colorant ou le second composé si le premier composé est un composé colorant) peut être un composé destiné à être coloré. La coloration obtenue est plus stable que celle obtenue avec les procédés de l'art antérieur, du fait que, à l'issue du procédé, le composé colorant est lié de manière covalente (via un groupe imine) au composé destiné à être coloré.

Plus spécifiquement, le premier composé peut être un composé destiné à être coloré et le second composé peut être un composé colorant, ledit procédé de l'invention pouvant ainsi être un procédé de coloration.

Le premier composé peut être tout composé comportant au moins un groupe amine et, en particulier, lorsque le procédé consiste en un procédé de coloration, le premier composé peut être un polymère comprenant, au niveau de tout ou partie de ses motifs répétitifs, au moins un groupe amine. Plus spécifiquement, il peut s'agir d'un polymère appartenant à la famille des chitosanes, c'est-à-dire des polyosides résultant de la distribution aléatoire d'unités D-glucosamine liées en ß-(1-4) et d'unités N-acétyl-D-glucosamine. Les chitosanes constituent des biopolymères biodégradables.

Dans ce cas de figure, le premier composé peut être intégré dans une feuille de papier (à hauteur, par exemple, de 20% massique), le procédé consistant ainsi, lorsque le second composé est un composé colorant, en un procédé d'obtention de papier coloré par coloration de feuille(s) à base de chitosane.

Le second composé peut être tout composé comportant au moins un groupe carbonyle, de préférence, aldéhyde et, en particulier, lorsque le procédé consiste en un procédé de coloration, le second composé est, avantageusement, un composé colorant (ou en d'autres termes, un composé comprenant au moins un groupe chromophore), ce qui signifie que le second composé, en réagissant *via* un groupe carbonyle avec un groupe amine du premier composé pour former un groupe imine, va conférer une couleur au premier composé.

Plus spécifiquement, le second composé peut être un composé comprenant un ou plusieurs cycles aromatiques, le ou tout ou partie desdits cycles aromatiques comprenant :
- au moins un groupe carbonyle ; et
- éventuellement au moins un autre groupe différent d'un groupe carbonyle et comprenant au moins un atome porteur d'un doublet libre, ce qui couvrent les situations suivantes :
   - le composé comprend un seul cycle aromatique, par exemple un cycle aromatique carboné (c'est-à-dire dont tous les atomes constitutifs du cycle sont des atomes de carbone) ou comprenant au moins un hétéroatome (le cycle aromatique pouvant être ainsi qualifié de cycle hétéroaromatique), ledit cycle aromatique comprenant au moins un groupe carbonyle et éventuellement au moins un autre groupe différent d'un groupe carbonyle et comprenant au moins un atome porteur d'un doublet libre;
   - le composé comprend plusieurs cycles aromatiques, tout ou partie desdits cycles étant des cycles aromatiques carbonés (c'est-à-dire dont tous les atomes constitutifs du ou des cycles sont des atomes de carbone) ou comprenant au moins un hétéroatome et comprenant au moins un groupe carbonyle et tout ou partie desdits cycles comprenant éventuellement au moins un autre groupe différent d'un groupe carbonyle et comprenant au moins un atome porteur d'un doublet libre.

Le second composé peut être un composé aromatique comprenant un seul cycle aromatique, lequel cycle aromatique étant porteur d'au moins un groupe carbonyle et d'au moins un autre groupe différent d'un groupe carbonyle et comprenant au moins un atome porteur d'un doublet libre.

Concernant l'autre groupe différent d'un groupe carbonyle et comprenant au moins un atome porteur d'un doublet libre, il peut s'agir d'un groupe comprenant un atome d'oxygène, un atome de soufre et/ou un atome d'azote, de préférence, un groupe attracteur d'électrons et à effet mésomère donneur, tel qu'un groupe comprenant un atome d'oxygène, par exemple, un groupe hydroxyle.

Le second composé peut consister en un composé monocyclique aromatique carboné (ce qui signifie que tous les atomes constitutifs du cycle sont des atomes de carbone), par exemple, à six chaînons (tel qu'un groupe phényle), étant entendu, dans ce cas, que ce cycle est porteur à la fois d'au moins un groupe carbonyle et d'au moins un autre groupe différent d'un groupe carbonyle et comprenant au moins un atome porteur d'un doublet libre (par exemple, un atome d'oxygène, un atome de soufre et/ou un atome d'azote), tel qu'un groupe attracteur d'électrons et à effet mésomère donneur, par exemple comprenant un atome d'oxygène, tel qu'un groupe hydroxyle.

Plus spécifiquement, le second composé peut consister en un composé monocyclique aromatique carboné (c'est-à-dire dont tous les atomes constitutifs du cycle sont des atomes de carbone), tel qu'un groupe phényle, porteur d'un seul groupe carbonyle et d'un seul autre groupe différent d'un groupe carbonyle et comprenant au moins un atome porteur d'un doublet libre (par exemple, un atome d'oxygène, un atome de soufre et/ou un atome d'azote), tel qu'un groupe attracteur d'électrons et à effet mésomère donneur, par exemple comprenant un atome d'oxygène, tel qu'un groupe hydroxyle, ledit groupe carbonyle et l'autre groupe étant en position *ortho* l'un de l'autre sur le cycle aromatique carboné. Ce type de composé est apte à dissiper l'énergie absorbée par rotation et permet après réaction avec un premier composé pour former un groupe imine de conférer une couleur au premier composé présentant une bonne tenue à la lumière.

En outre, le cycle aromatique peut comprendre un ou plusieurs autres groupes, que ceux mentionnés ci-dessus, par exemple, des groupes choisis parmi les atomes d'halogène, les groupes alkyles, les groupes alcoxy, les groupes esters.

A titre d'exemple de second composé, il peut être fait mention d'un composé répondant à la formule (I) suivante : 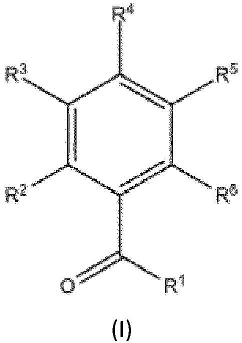 dans laquelle :
- R¹ représente un atome d'hydrogène ou un groupe alkyle, de préférence, un atome d'hydrogène ;
- R² représente un groupe différent d'un groupe carbonyle et comprenant un atome d'oxygène attracteur d'électrons et à effet mésomère donneur, tel qu'un groupe hydroxyle ;
- R³ à R⁶ représentent, indépendamment l'un de l'autre, un atome d'hydrogène, un groupe alkyle, un groupe alcoxy, un groupe ester.

Plus spécifiquement, l'un au moins des groupes R³ à R⁶ est un groupe ester et, encore plus spécifiquement, un seul des groupes R³ à R⁶ est un groupe ester, les autres groupes étant un atome d'hydrogène.

Un composé répondant à ces spécificités peut être un groupe de formule (II) suivante : 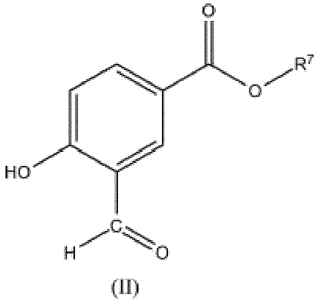 dans laquelle R⁷ représente un groupe alkyle, tel qu'un groupe éthyle.

Lorsque ce composé de formule (II) est mis à réagir avec un premier composé de la famille des chitosanes, lequel est intégré dans une feuille de papier (par exemple, à hauteur de 20% massique), la feuille de papier affiche, après réaction, une couleur jaune intense et montrant une forte fluorescence sous éclairage UV (365 nm).

Selon un mode de réalisation, le second composé est un composé aromatique comprenant plusieurs cycles aromatiques, par exemple, deux cycles aromatiques, lesdits cycles aromatiques étant accolés, l'un au moins desdits cycles comprenant au moins un groupe carbonyle et l'un au moins desdits cycles comprenant au moins un autre groupe différent d'un groupe carbonyle et comprenant au moins un atome porteur d'un doublet libre.

Concernant l'autre groupe, il peut s'agir d'un groupe comprenant un atome d'oxygène, un atome de soufre et/ou un atome d'azote, de préférence, un groupe attracteur d'électrons et à effet mésomère donneur, tel qu'un groupe comprenant un atome d'oxygène, par exemple, un groupe hydroxyle.

En particulier, le second composé peut consister en un composé bicyclique aromatique, dont les cycles sont accolés, l'un des cycles étant un cycle hétéroaromatique, tandis que l'autre cycle est un cycle aromatique carboné (c'est-à-dire dont tous les atomes constitutifs du cycle sont des atomes de carbone), par exemple, à 6 chaînons (tel qu'un groupe phényle), l'un des cycles étant porteur d'au moins un groupe carbonyle et l'un des cycles étant porteur d'au moins un autre groupe différent d'un groupe carbonyle et comprenant au moins un atome porteur d'un doublet libre (par exemple, un atome d'oxygène, un atome de soufre et/ou un atome d'azote), tel qu'un groupe attracteur d'électrons et à effet mésomère donneur, par exemple comprenant un atome d'oxygène, tel qu'un groupe hydroxyle.

Plus spécifiquement, le second composé peut consister en un composé bicyclique aromatique, dont les cycles sont accolés, l'un des cycles étant un cycle hétéroaromatique, tandis que l'autre cycle est un cycle aromatique carboné (ce qui signifie que tous les atomes constitutifs du cycle sont des atomes de carbone), par exemple, à 6 chaînons (tel qu'un groupe phényle), le cycle hétéroaromatique étant porteur d'au moins un groupe carbonyle et le cycle aromatique carboné étant porteur d'au moins un autre groupe différent d'un groupe carbonyle et comprenant au moins un atome porteur d'un doublet libre (par exemple, un atome d'oxygène, un atome de soufre et/ou un atome d'azote), tel qu'un groupe attracteur d'électrons et à effet mésomère donneur, par exemple comprenant un atome d'oxygène, tel qu'un groupe hydroxyle.

A titre d'exemple de second composé, il peut être fait mention d'un groupe répondant à la formule (III) suivante : 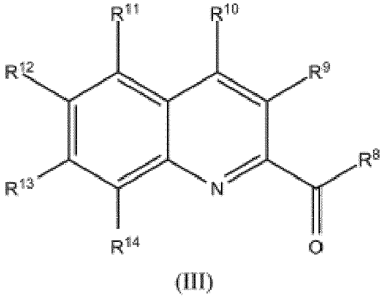 dans laquelle :
- R⁸ représente un atome d'hydrogène ou un groupe alkyle, de préférence, un atome d'hydrogène ;
- R¹⁴ représente un groupe différent d'un groupe carbonyle et comprenant un atome d'oxygène attracteur d'électrons et à effet mésomère donneur, tel qu'un groupe hydroxyle ;
- R⁹ à R¹³ représentent, indépendamment l'un de l'autre, un atome d'hydrogène, un groupe alkyle, un groupe alcoxy, un groupe ester.

Un composé spécifique répondant à ces spécificités peut être un groupe de formule (IV) suivante : 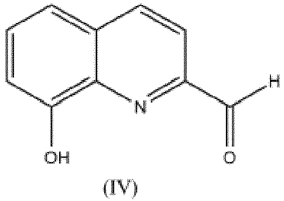 ce composé correspondant au 8-hydroxy-2-quinolinecarboxaldéhyde.

Par ailleurs, au moins l'un des premier ou second composé peut présenter une température de fusion inférieure à la température de mise en oeuvre de l'étape de réaction et, plus spécifiquement, au moins l'un des premier ou second composé peut présenter une température de fusion inférieure à 120°C ou encore inférieure ou égale à 100°C. Dans de telles conditions, au moins l'un des premier ou second composés se trouve à l'état liquide dans les conditions opératoires de l'étape de réaction (ces conditions opératoires étant des conditions nécessaires pour que le fluide soit dans des conditions supercritiques), ce qui permet un meilleur transport et une meilleure activation des composés pour engendrer la réaction de condensation.

Plus précisément, le second composé présente une température de fusion inférieure à la température de mise en oeuvre de la réaction en présence d'au moins un fluide supercritique et, plus spécifiquement, la température de fusion du second composé peut être inférieure à 120°C ou encore inférieure ou égale à 100°C, ce qui lui permet d'être à l'état liquide dans les conditions opératoires de l'étape de réaction, ce qui permet une meilleure vectorisation de celui-ci vers le premier composé et donc de faciliter la réaction de condensation entre le premier composé et le second composé.

Parmi les composés obtenus selon le procédé de l'invention, certains sont nouveaux et, en particulier, les composés susceptibles d'être obtenus par le procédé de l'invention et étant des composés de la famille des chitosanes fonctionnalisés par des groupes, dits groupes A, comprenant un ou plusieurs cycles aromatiques, le ou tout ou partie desdits cycles aromatiques comprenant au moins un groupe imine et le ou tout ou partie desdits cycles comprenant au moins un autre groupe différent d'un groupe imine et comprenant au moins un atome porteur d'un doublet libre.

On précise, par «fonctionnalisé», que l'on entend, au sens de l'invention, une fixation des groupes susmentionnés sur les composés par le biais de liaisons covalentes.

Comme mentionné ci-dessus, ces composés appartiennent à la famille des chitosanes, c'est-à-dire des polyosides résultant de la distribution aléatoire d'unités D-glucosamine liées en ß-(1-4) et d'unités N-acétyl-D-glucosamine, tout ou partie des unités D-glucosamine ayant leurs groupes amines transformés en groupes imines, lesdits groupes imines étant ceux appartenant aux groupes A, ce qui signifie en d'autres termes, que les groupes A sont liés aux polymères chitosanes au niveau des positions initialement occupées par ses groupes amines, lesquels sont convertis en groupes imines. Dans ce cas particulier, le polymère concerné constitue un reste de chitosane, du fait que les groupes amines sont en tout ou partie convertis en groupes imines, lesquels groupes imines font partie désormais des groupes A.

Selon un premier mode de réalisation, les groupes A consistent en des groupes aromatiques comprenant un seul cycle aromatique, lequel cycle aromatique étant porteur d'au moins un groupe imine et d'au moins un autre groupe différent d'un groupe imine et comprenant au moins un atome porteur d'un doublet libre.

Concernant l'autre groupe, il peut s'agir d'un groupe comprenant un atome d'oxygène, un atome de soufre et/ou un atome d'azote, de préférence, un groupe attracteur d'électrons et à effet mésomère donneur, tel qu'un substituant comprenant un atome d'oxygène, par exemple, un substituant hydroxyle.

En particulier, les groupes A peuvent consister en des groupes monocycliques aromatiques carbonés (ce qui signifie que tous les atomes constitutifs des cycles sont des atomes de carbone), par exemple, à 6 chaînons (tel qu'un groupe phényle), étant entendu, dans ce cas, que le cycle des groupes est porteur à la fois d'au moins un groupe imine et d'au moins un autre groupe différent d'un groupe imine et comprenant au moins un atome porteur d'un doublet libre (par exemple, un atome d'oxygène, un atome de soufre et/ou un atome d'azote), tel qu'un groupe attracteur d'électrons et à effet mésomère donneur, par exemple comprenant un atome d'oxygène, tel qu'un groupe hydroxyle.

Plus spécifiquement, les groupes A peuvent consister en des groupes monocycliques aromatiques carbonés, par exemple, des groupes phényles, porteurs d'un seul groupe imine et d'un seul autre groupe différent d'un groupe imine et comprenant au moins un atome porteur d'un doublet libre (par exemple, un atome d'oxygène, un atome de soufre et/ou un atome d'azote), tel qu'un groupe attracteur d'électrons et à effet mésomère donneur, par exemple comprenant un atome d'oxygène, tel qu'un groupe hydroxyle, ledit groupe imine et l'autre groupe étant en position *ortho* l'un de l'autre sur le cycle aromatique.

En outre, le cycle aromatique constitutif de ces groupes peut comprendre un ou plusieurs autres groupes, tels que ceux mentionnés ci-dessus, par exemple, des groupes choisis parmi les atomes d'halogène, les groupes alkyles, les groupes alcoxy, les groupes esters.

A titre d'exemple de groupes A, il peut être fait mention de groupes répondant à la formule (V) suivante : 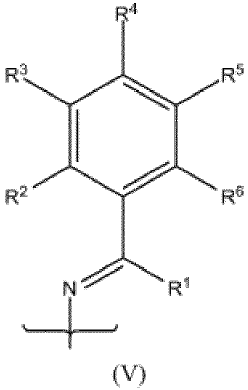 dans laquelle :
- R¹ représente un atome d'hydrogène ou un groupe alkyle, de préférence, un atome d'hydrogène ;
- R² représente un groupe comprenant un atome d'oxygène attracteur d'électrons et à effet mésomère donneur, tel qu'un groupe hydroxyle ;
- R³ à R⁶ représentent, indépendamment l'un de l'autre, un atome d'hydrogène, un groupe alkyle, un groupe alcoxy, un groupe ester,
l'accolade indiquant l'endroit par lequel le groupe A est lié au reste de chitosane soit directement (ce qui signifie que l'atome d'azote est directement par covalence au reste de chitosane) soit *via* un groupe espaceur organique (ce qui signifie que le groupe espaceur organique forme pont entre l'atome d'azote et le reste de chitosane), ce groupe espaceur organique pouvant être un groupe alkylène.

Plus spécifiquement, l'un au moins des groupes R³ à R⁶ est un groupe ester et, encore plus spécifiquement, un seul des groupes R³ à R⁶ est un groupe ester, les autres groupes étant un atome d'hydrogène.

Un groupe A répondant à ces spécificités peut être un groupe de formule (VI) suivante : 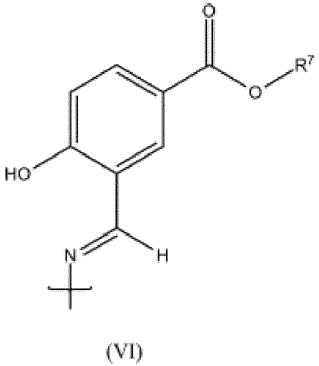 dans laquelle R⁷ représente un groupe alkyle et l'accolade indiquant l'endroit par lequel le groupe A est lié au reste de chitosane soit directement (ce qui signifie que l'atome d'azote est directement par covalence au reste de chitosane) soit *via* un groupe espaceur organique (ce qui signifie que le groupe espaceur organique forme pont entre l'atome d'azote et le reste de chitosane).

Selon un deuxième mode de réalisation, les groupes A peuvent consister en des groupes aromatiques comprenant plusieurs cycles aromatiques, par exemple, deux cycles aromatiques, lesdits cycles aromatiques étant accolés, l'un au moins desdits cycles comprenant au moins un groupe imine et l'un au moins desdits cycles comprenant au moins un autre groupe différent d'un groupe imine et comprenant au moins un atome porteur d'un doublet libre.

Concernant l'autre groupe, il peut s'agir d'un groupe comprenant un atome d'oxygène, un atome de soufre et/ou un atome d'azote, de préférence, un groupe attracteur d'électrons et à effet mésomère donneur, tel qu'un groupe comprenant un atome d'oxygène, par exemple, un groupe hydroxyle.

En particulier, les groupes A peuvent consister en des groupes bicycliques aromatiques, dont les cycles sont accolés, l'un des cycles étant un cycle hétéroaromatique, tandis que l'autre cycle est un cycle aromatique carboné (c'est-à-dire dont tous les atomes constitutifs du cycle sont des atomes de carbone), par exemple, à 6 chaînons (tel qu'un groupe phényle), étant entendu, que l'un des cycles est porteur d'au moins un groupe imine et l'un des cycles est porteur d'au moins un autre groupe différent d'un groupe imine et comprenant au moins un atome porteur d'un doublet libre (par exemple, un atome d'oxygène, un atome de soufre et/ou un atome d'azote), tel qu'un groupe attracteur d'électrons et à effet mésomère donneur, par exemple comprenant un atome d'oxygène, tel qu'un groupe hydroxyle.

Plus spécifiquement, les groupes A peuvent consister en des groupes bicycliques aromatiques, dont les cycles sont accolés, l'un des cycles étant un cycle hétéroaromatique, tandis que l'autre cycle est un cycle aromatique carboné (ce qui signifie que tous les atomes constitutifs du cycle sont des atomes de carbone), par exemple, à 6 chaînons (tel qu'un groupe phényle), le cycle hétéroaromatique étant porteur d'au moins un groupe imine et le cycle aromatique carboné étant porteur d'au moins un autre groupe différent d'un groupe imine et comprenant au moins un atome porteur d'un doublet libre (par exemple, un atome d'oxygène, un atome de soufre et/ou un atome d'azote), tel qu'un groupe attracteur d'électrons et à effet mésomère donneur, par exemple comprenant un atome d'oxygène, tel qu'un groupe hydroxyle.

A titre d'exemple de groupes A, il peut être fait mention d'un groupe répondant à la formule (VII) suivante : 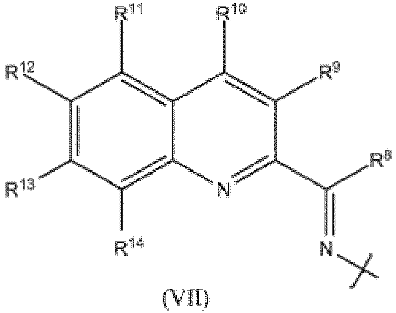 dans laquelle :
- R⁸ représente un atome d'hydrogène ou un groupe alkyle, de préférence, un atome d'hydrogène ;
- R¹⁴ représente un groupe comprenant un atome d'oxygène attracteur d'électrons et à effet mésomère donneur, tel qu'un groupe hydroxyle ;
- R⁹ à R¹³ représentent, indépendamment l'un de l'autre, un atome d'hydrogène, un groupe alkyle, un groupe alcoxy, un groupe ester,
l'accolade indiquant l'endroit par lequel le groupe A est lié au reste de chitosane soit directement (ce qui signifie que l'atome d'azote est directement par covalence au reste de chitosane) soit *via* un groupe espaceur organique (ce qui signifie que le groupe espaceur organique forme pont entre l'atome d'azote et le reste de chitosane).

Un groupe A spécifique répondant à ces spécificités est un groupe de formule (VIII) suivante :

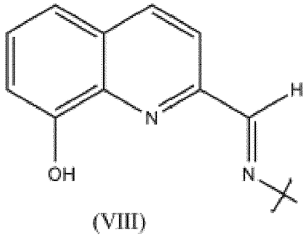

Les composés appartenant à la famille des chitosanes conformes à l'invention peuvent être avantageusement incorporés dans du papier pour lui conférer une couleur stable dans le temps et qui n'est pas susceptible de subir un lessivage après mise en contact avec de l'humidité ou un solvant organique.

Ainsi, l'invention a trait également à un papier comprenant au moins un composé appartenant à la famille des chitosanes conforme à l'invention, ce composé pouvant être présent à hauteur d'au moins 20% massique par rapport à la masse totale du papier.

D'autres caractéristiques et avantages de l'invention apparaîtront du complément de description qui suit qui se rapporte à deux exemples de préparation.

Bien entendu, ce complément de description n'est donné qu'à titre d'illustration de l'invention et n'en constitue en aucun cas une limitation.

### EXPOSÉ DÉTAILLÉ DE MODES DE RÉALISATION PARTICULIERS

### EXEMPLE 1

Cet exemple illustre un procédé conforme à l'invention et, plus spécifiquement, un procédé de coloration d'un papier.

Le papier concerné est un papier spécifique comprenant un chitosane à hauteur de 20% massique, qui est un polymère naturel présentant des groupes pendants amine primaire, lesquels groupes sont très réactifs pour réagir avec des groupes aldéhydes et former des groupes imines.

Le papier susmentionné est placé dans un réacteur rempli de CO₂ supercritique et d'un composé de formule (II) dans lequel R⁷ est un groupe éthyle. 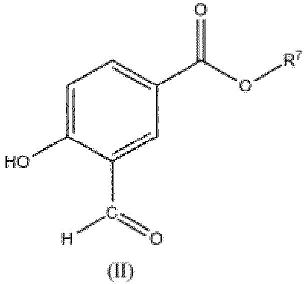 ce composé correspondant à l'éthyl 3-formyl-4-hydroxybenzoate.

La pression est fixée à 280 bars et la température est fixée à 105°C moyennant quoi le CO₂ se trouve à l'état supercritique. Dans ces conditions, l'éthyl 3-formyl-4-hydroxybenzoate est sous forme liquide sachant que son point de fusion est de 67-73°C et est soluble dans le CO₂ supercritique compte tenu de son caractère lipophile dû à la présence d'un groupe benzoate d'éthyle. La pression et la température susmentionnées sont maintenues pendant 1 heure.

A l'issue de cette durée, le papier résultant affiche une couleur jaune intense.

Afin de confirmer l'efficacité du procédé, en parallèle, la même réaction a été conduite avec un échantillon de papier identique et le même aldéhyde mais dans l'éthanol. Il aura fallu 2 semaines par voie humide pour conduire cette réaction de coloration à terme, mettant ainsi en évidence le gain de temps considérable pour reproduire cette réaction sous CO₂ supercritique.

Des tests normés (ISO 105-B02) de tenue à la lumière et de tenue de la couleur ont été menés afin d'évaluer la performance colorimétrique de l'échantillon coloré obtenu sous CO₂ supercritique. Ainsi la tenue lumière sous UV de l'échantillon a été évaluée à 3. Pour les fabricants de papiers, les substrats cellulosiques sont considérés comme ayant une tenue lumière convenable à partir de 3. La tenue de la couleur, quant à elle, a été évaluée à 3-4, ce qui est satisfaisant.

Enfin, afin de confirmer l'accroche covalente du colorant sur le papier, l'échantillon coloré a été soumis à un nettoyage puis à des lessivages à l'acétone et à l'éthanol. Après séchage à température ambiante, l'échantillon conserve une bonne teinte jaune. La confirmation de l'accroche covalente du colorant *via* la formation d'une fonction imine (C=N) a été validée par spectroscopie IR, avec observation de la bande d'étirement de la fonction C=N à 1635 cm⁻¹, bande absente sur le papier d'origine.

### EXEMPLE 2

Cet exemple illustre un procédé conforme à l'invention et un composé obtenu conformément au procédé de l'invention et conforme à l'invention, ce composé résultant de la condensation d'un polymère chitosane avec un composé de formule (IV) suivante :

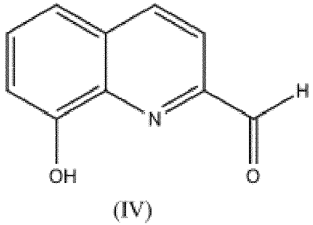

Ces deux réactifs sont placés dans un réacteur rempli de CO₂, La pression étant fixée à 260 bars et la température est fixée à 105°C, moyennant quoi le CO₂ se trouve à l'état supercritique. Dans ces conditions, le 8-hydroxy-2-quinolinecarboxaldéhyde est sous forme liquide sachant que son point de fusion est de 87-100 °C. La pression et la température susmentionnées sont maintenues pendant 3 heures.

A l'issue de cette durée, le chitosane initialement incolore affiche une teinte orangée, typique de l'augmentation du système conjugué et preuve que le chitosane et le 8-hydroxy-2-quinolinecarboxaldéhyde ont réagi ensemble de manière covalente.

## Revendications

1. Procédé de fabrication d'un composé comprenant au moins un groupe imine, ledit procédé comprenant une étape de réaction entre un premier composé comprenant au moins un groupe amine et un second composé comprenant au moins un groupe carbonyle, ladite étape de réaction étant réalisée en présence d'au moins un fluide supercritique.

2. Procédé selon la revendication 1, dans lequel l'étape de réaction est réalisée en présence d'un seul fluide supercritique, qui est du CO₂ supercritique.

3. Procédé selon l'une quelconque des revendications précédentes, dans lequel le premier composé est un polymère comprenant, au niveau de tout ou partie de ses motifs répétitifs, au moins un groupe amine.

4. Procédé selon l'une quelconque des revendications précédentes, dans lequel le second composé est un composé comprenant un ou plusieurs cycles aromatiques, le ou tout ou partie desdits cycles aromatiques comprenant au moins un groupe carbonyle et éventuellement au moins un autre groupe différent d'un groupe carbonyle et comprenant au moins un atome porteur d'un doublet libre.

5. Procédé selon l'une quelconque des revendications précédentes, dans lequel le second composé est un composé monocyclique aromatique carboné porteur d'un seul groupe carbonyle et d'un seul autre groupe différent d'un groupe carbonyle et comprenant au moins un atome porteur d'un doublet libre, tel qu'un groupe attracteur d'électrons et à effet mésomère donneur, par exemple comprenant un atome d'oxygène, tel qu'un groupe hydroxyle, ledit groupe carbonyle et l'autre groupe étant en position *ortho* l'un de l'autre sur le cycle aromatique carboné.

6. Procédé selon l'une quelconque des revendications précédentes, dans lequel le second composé est un composé répondant à la formule (I) suivante : 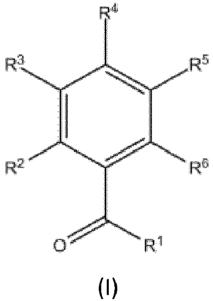 dans laquelle :
- R¹ représente un atome d'hydrogène ou un groupe alkyle ;
- R² représente un groupe différent d'un groupe carbonyle et comprenant un atome d'oxygène attracteur d'électrons et à effet mésomère donneur, tel qu'un groupe hydroxyle ;
- R³ à R⁶ représentent, indépendamment l'un de l'autre, un atome d'hydrogène, un groupe alkyle, un groupe alcoxy, un groupe ester.

7. Procédé selon l'une quelconque des revendications 1 à 4, dans lequel le second composé est un composé aromatique comprenant plusieurs cycles aromatiques, lesdits cycles aromatiques étant accolés, l'un au moins desdits cycles comprenant au moins un groupe carbonyle et l'un au moins desdits cycles comprenant au moins un autre groupe différent d'un groupe carbonyle et comprenant au moins un atome porteur d'un doublet libre.

8. Procédé selon l'une quelconque des revendications 1 à 4 ou 7, dans lequel le second composé est un composé bicyclique aromatique, dont les cycles sont accolés, l'un des cycles étant un cycle hétéroaromatique, tandis que l'autre cycle est un cycle aromatique carboné, l'un des cycles étant porteur d'au moins un groupe carbonyle et l'un des cycles étant porteur d'au moins un autre groupe différent d'un groupe carbonyle et comprenant au moins un atome porteur d'un doublet libre, tel qu'un groupe attracteur d'électrons et à effet mésomère donneur, par exemple comprenant un atome d'oxygène, tel qu'un groupe hydroxyle.

9. Procédé selon l'une quelconque des revendications 1 à 4, 7, ou 8, dans lequel le second composé répond à la formule (III) suivante : 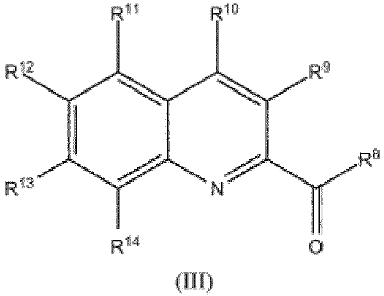 dans laquelle :
- R⁸ représente un atome d'hydrogène ou un groupe alkyle, de préférence, un atome d'hydrogène ;
- R¹⁴ représente un groupe différent d'un groupe carbonyle et comprenant un atome d'oxygène attracteur d'électrons et à effet mésomère donneur ;
- R⁹ à R¹³ représentent, indépendamment l'un de l'autre, un atome d'hydrogène, un groupe alkyle, un groupe alcoxy, un groupe ester.

10. Composé susceptible d'être obtenu par le procédé tel que défini à la revendication 1, qui est un composé de la famille des chitosanes fonctionnalisés par des groupes, dits groupes A, comprenant un ou plusieurs cycles aromatiques, le ou tout ou partie desdits cycles aromatiques comprenant au moins un groupe imine et le ou tout ou partie desdits cycles comprenant au moins un autre groupe différent d'un groupe imine et comprenant au moins un atome porteur d'un doublet libre.

11. Composé selon la revendication 10, dans lequel les groupes A consistent en des groupes aromatiques comprenant un seul cycle aromatique, lequel cycle aromatique étant porteur d'au moins un groupe imine et d'au moins un autre groupe différent d'un groupe imine et comprenant au moins un atome porteur d'un doublet libre.

12. Composé selon l'une quelconque des revendications 10 ou 11, dans lequel les groupes A répondent à la formule (V) suivante : 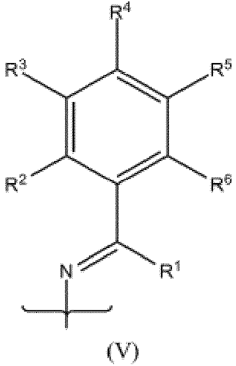 dans laquelle :
- R¹ représente un atome d'hydrogène ou un groupe alkyle ;
- R² représente un groupe comprenant un atome d'oxygène attracteur d'électrons et à effet mésomère donneur ;
- R³ à R⁶ représentent, indépendamment l'un de l'autre, un atome d'hydrogène, un groupe alkyle, un groupe alcoxy, un groupe ester,
l'accolade indiquant l'endroit par lequel le groupe A est lié au reste de chitosane soit directement soit *via* un groupe espaceur organique.

13. Composé selon la revendication 10, dans lequel les groupes A consistent en des groupes aromatiques comprenant plusieurs cycles aromatiques, lesdits cycles aromatiques étant accolés, l'un au moins desdits cycles comprenant au moins un groupe imine et l'un au moins desdits cycles comprenant au moins un autre groupe différent d'un groupe imine et comprenant au moins un atome porteur d'un doublet libre.

14. Composé, selon la revendication 13, dans lequel les groupes A répondent à la formule (VII) suivante : 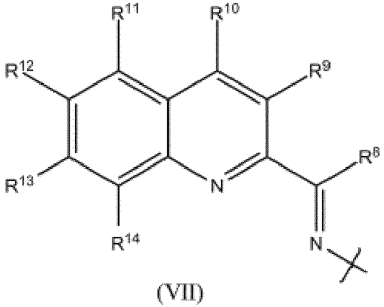 dans laquelle :
- R⁸ représente un atome d'hydrogène ou un groupe alkyle ;
- R¹⁴ représente un groupe comprenant un atome d'oxygène attracteur d'électrons et à effet mésomère donneur ;
- R⁹ à R¹³ représentent, indépendamment l'un de l'autre, un atome d'hydrogène, un groupe alkyle, un groupe alcoxy, un groupe ester,
l'accolade indiquant l'endroit par lequel le groupe A est lié au reste de chitosane soit directement soit *via* un groupe espaceur organique.

15. Papier comprenant au moins un composé appartenant à la famille des chitosanes tel que défini selon l'une quelconque des revendications 10 à 14.

## Patentansprüche

1. Verfahren zur Herstellung einer Verbindung, die mindestens eine Imingruppe umfasst, wobei das Verfahren einen Reaktionsschritt zwischen einer ersten Verbindung, die mindestens eine Aminogruppe umfasst, und einer zweiten Verbindung umfasst, die mindestens eine Carbonylgruppe umfasst, wobei der Reaktionsschritt in Gegenwart mindestens eines überkritischen Fluids ausgeführt wird.

2. Verfahren nach Anspruch 1, wobei der Reaktionsschritt in Gegenwart eines einzigen überkritischen Fluids ausgeführt wird, bei dem es sich um überkritisches CO₂ handelt.

3. Verfahren nach einem der vorstehenden Ansprüche, wobei die erste Verbindung ein Polymer ist, das an allen oder einem Teil seiner Wiederholungseinheiten mindestens eine Aminogruppe umfasst.

4. Verfahren nach einem der vorstehenden Ansprüche, wobei die zweite Verbindung eine Verbindung ist, die einen oder mehrere aromatische Ringe umfasst, wobei der oder alle oder ein Teil der aromatischen Ringe mindestens eine Carbonylgruppe und gegebenenfalls mindestens eine andere Gruppe umfassen, die sich von einer Carbonylgruppe unterscheidet und mindestens ein Atom umfasst, das ein freies Elektronenpaar trägt.

5. Verfahren nach einem der vorstehenden Ansprüche, wobei die zweite Verbindung eine monocyclische aromatische Kohlenstoffverbindung ist, die eine einzige Carbonylgruppe und eine einzige andere Gruppe trägt, die sich von einer Carbonylgruppe unterscheidet und mindestens ein Atom umfasst, das ein freies Elektronenpaar trägt, wie etwa eine elektronenziehende und mesomeren Donatoreffekt besitzende Gruppe, die zum Beispiel ein Sauerstoffatom umfasst, wie etwa eine Hydroxylgruppe, wobei sich die Carbonylgruppe und die andere Gruppe am aromatischen Kohlenstoffring in ortho-Stellung zueinander befinden.

6. Verfahren nach einem der vorstehenden Ansprüche, wobei die zweite Verbindung eine Verbindung ist, die der folgenden Formel (I) entspricht: 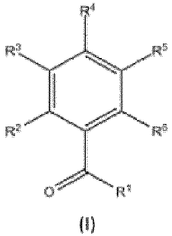 wobei:
- R¹ ein Wasserstoffatom oder eine Alkylgruppe darstellt;
- R² eine Gruppe darstellt, die sich von einer Carbonylgruppe unterscheidet und ein elektronenziehendes und mesomeren Donatoreffekt besitzendes Sauerstoffatom umfasst, wie etwa eine Hydroxylgruppe;
- R³ bis R⁶ unabhängig voneinander ein Wasserstoffatom, eine Alkylgruppe, eine Alkoxygruppe, eine Estergruppe darstellen.

7. Verfahren nach einem der Ansprüche 1 bis 4, wobei die zweite Verbindung eine aromatische Verbindung ist, die mehrere aromatische Ringe umfasst, wobei die aromatischen Ringe kondensiert sind, wobei mindestens einer der Ringe mindestens eine Carbonylgruppe umfasst und mindestens einer der Ringe mindestens eine andere Gruppe umfasst, die sich von einer Carbonylgruppe unterscheidet und mindestens ein Atom umfasst, das ein freies Elektronenpaar trägt.

8. Verfahren nach einem der Ansprüche 1 bis 4 oder 7, wobei die zweite Verbindung eine bicyclische aromatische Verbindung ist, deren Ringe kondensiert sind, wobei einer der Ringe ein heteroaromatischer Ring ist, während der andere Ring ein aromatischer Kohlenstoffring ist, wobei einer der Ringe mindestens eine Carbonylgruppe trägt und einer der Ringe mindestens eine andere Gruppe trägt, die sich von einer Carbonylgruppe unterscheidet und mindestens ein Atom umfasst, das ein freies Elektronenpaar trägt, wie etwa eine elektronenziehende und mesomeren Donatoreffekt besitzende Gruppe, die zum Beispiel ein Sauerstoffatom umfasst, wie etwa eine Hydroxylgruppe.

9. Verfahren nach einem der Ansprüche 1 bis 4, 7 oder 8, wobei die zweite Verbindung der folgenden Formel (III) entspricht: 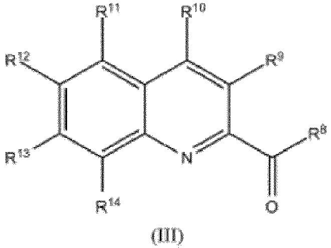 wobei:
- R⁸ ein Wasserstoffatom oder eine Alkylgruppe, bevorzugt ein Wasserstoffatom, darstellt;
- R¹⁴ eine Gruppe darstellt, die sich von einer Carbonylgruppe unterscheidet und ein elektronenziehendes und mesomeren Donatoreffekt besitzendes Sauerstoffatom umfasst;
- R⁹ bis R¹³ unabhängig voneinander ein Wasserstoffatom, eine Alkylgruppe, eine Alkoxygruppe, eine Estergruppe darstellen.

10. Verbindung, die durch das Verfahren, wie es in Anspruch 1 definiert ist, erhalten werden kann, bei der es sich um eine Verbindung der Familie der Chitosane handelt, die mit als A-Gruppen bezeichneten Gruppen funktionalisiert ist, welche einen oder mehrere aromatische Ringe umfassen, wobei der oder alle oder ein Teil der aromatischen Ringe mindestens eine Imingruppe umfassen und der oder alle oder ein Teil der Ringe mindestens eine andere Gruppe umfassen, die sich von einer Imingruppe unterscheidet und mindestens ein Atom umfasst, das ein freies Elektronenpaar trägt.

11. Verbindung nach Anspruch 10, wobei die A-Gruppen aus aromatischen Gruppen bestehen, die einen einzigen aromatischen Ring umfassen, wobei der aromatische Ring mindestens eine Imingruppe und mindestens eine andere Gruppe trägt, die sich von einer Imingruppe unterscheidet und mindestens ein Atom umfasst, das ein freies Elektronenpaar trägt.

12. Verbindung nach einem der Ansprüche 10 oder 11, wobei die A-Gruppen der folgenden Formel (V) entsprechen: 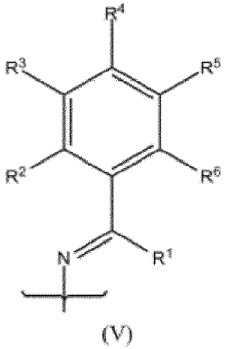 wobei:
- R¹ ein Wasserstoffatom oder eine Alkylgruppe darstellt;
- R² eine Gruppe darstellt, die ein elektronenziehendes und mesomeren Donatoreffekt besitzendes Sauerstoffatom umfasst;
- R³ bis R⁶ unabhängig voneinander ein Wasserstoffatom, eine Alkylgruppe, eine Alkoxygruppe, eine Estergruppe darstellen,
wobei die geschweifte Klammer die Stelle angibt, an der die A-Gruppe entweder direkt oder über eine organische Spacergruppe an den Chitosanrest gebunden ist.

13. Verbindung nach Anspruch 10, wobei die A-Gruppen aus aromatischen Gruppen bestehen, die mehrere aromatische Ringe umfassen, wobei die aromatischen Ringe kondensiert sind, wobei mindestens einer der Ringe mindestens eine Imingruppe umfasst und mindestens einer der Ringe mindestens eine andere Gruppe umfasst, die sich von einer Imingruppe unterscheidet und mindestens ein Atom umfasst, das ein freies Elektronenpaar trägt.

14. Verbindung nach Anspruch 13, wobei die A-Gruppen der folgenden Formel (VII) entsprechen: 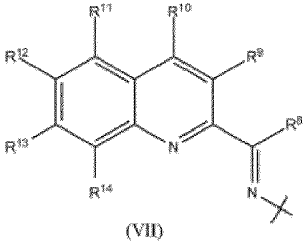 wobei:
- R⁸ ein Wasserstoffatom oder eine Alkylgruppe darstellt;
- R¹⁴ eine Gruppe darstellt, die ein elektronenziehendes und mesomeren Donatoreffekt besitzendes Sauerstoffatom umfasst;
- R⁹ bis R¹³ unabhängig voneinander ein Wasserstoffatom, eine Alkylgruppe, eine Alkoxygruppe, eine Estergruppe darstellen,
wobei die geschweifte Klammer die Stelle angibt, an der die A-Gruppe entweder direkt oder über eine organische Spacergruppe an den Chitosanrest gebunden ist.

15. Papier, das mindestens eine Verbindung umfasst, die zur Familie der Chitosane gehört, wie sie in einem der Ansprüche 10 bis 14 definiert ist.

## Claims

1. Method for manufacturing a compound comprising at least one imine group, said method comprising a step of reaction between a first compound comprising at least one amine group and a second compound comprising at least one carbonyl group, said reaction step being carried out in the presence of at least one supercritical fluid.

2. Method according to claim 1, wherein the reaction step is carried out in the presence of a single supercritical fluid, which is supercritical CO₂.

3. Method according to any one of the preceding claims, wherein the first compound is a polymer comprising, at all or part of the repeating units thereof, at least one amine group.

4. Method according to any one of the preceding claims, wherein the second compound is a compound comprising one or more aromatic rings, the or all or part of said aromatic rings comprising at least one carbonyl group and optionally at least one other group different from a carbonyl group and comprising at least one atom bearing a lone pair.

5. Method according to any one of the preceding claims, wherein the second compound is a monocyclic aromatic carbon compound bearing a single carbonyl group and a single other group different from a carbonyl group and comprising at least one atom bearing a lone pair, such as an electron withdrawing group and with a donor mesomeric effect, for example comprising an oxygen atom, such as a hydroxyl group, said carbonyl group and the other group being in the *ortho* position to each other on the aromatic carbon ring.

6. Method according to any one of the preceding claims, wherein the second compound is a compound having the following formula (I): 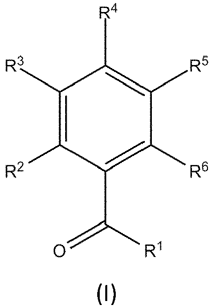 wherein:
- R¹ represents a hydrogen atom or an alkyl group;
- R² represents a group different from a carbonyl group and comprising an electron withdrawing oxygen atom and with a donor mesomeric effect, such as a hydroxyl group;
- R³ to R⁶ represent, independently of each other, a hydrogen atom, an alkyl group, an alkoxy group, an ester group.

7. Method according to any one of claims 1 to 4, wherein the second compound is an aromatic compound comprising several aromatic rings, said aromatic rings being fused, at least one of said rings comprising at least one carbonyl group and at least one of said rings comprising at least one other group different from a carbonyl group and comprising at least one atom bearing a lone pair.

8. Method according to any one of claims 1 to 4 or 7, wherein the second compound is a bicyclic aromatic compound, whose rings are fused, one of the rings being a heteroaromatic ring, while the other ring is an aromatic carbon ring, one of the rings is bearing at least one carbonyl group and one of the rings is bearing at least one other group different from a carbonyl group and comprising at least one atom bearing a lone pair, such as an electron withdrawing group and with a donor mesomeric effect, for example comprising an oxygen atom, such as a hydroxyl group.

9. Method according to any one of claims 1 to 4, 7 or 8, wherein the second compound has the following formula (III): 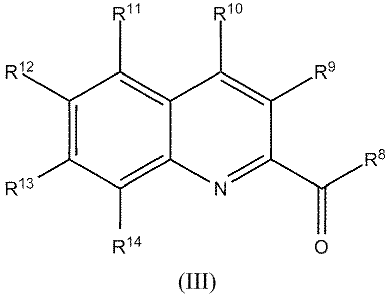 wherein:
- R⁸ represents a hydrogen atom or an alkyl group, preferably, a hydrogen atom;
- R¹⁴ represents a group different from a carbonyl group and comprising an electron withdrawing oxygen atom and with a donor mesomeric effect;
- R⁹ to R¹³ represent, independently of each other, a hydrogen atom, an alkyl group, an alkoxy group, an ester group.

10. Compound capable of being obtained by the method as defined in claim 1, which is a compound of the chitosan family functionalised by groups, called groups A, comprising one or more aromatic rings, the or all or part of said aromatic rings comprising at least one imine group and the or all or part of said rings comprising at least one other group different from an imine group and comprising at least one atom bearing a lone pair.

11. Compound according to claim 10, wherein the groups A consist of aromatic groups comprising a single aromatic ring, which aromatic ring is bearing at least one imine group and at least one other group different from an imine group and comprising at least one atom bearing a lone pair.

12. Compound according to claim 10 or 11, wherein the groups A have the following formula (V): 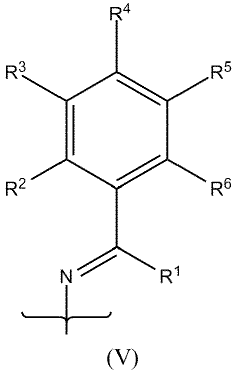 wherein:
- R¹ represents a hydrogen atom or an alkyl group;
- R² represents a group comprising an electron withdrawing oxygen atom and with a donor mesomeric effect;
- R³ to R⁶ represent, independently of each other, a hydrogen atom, an alkyl group, an alkoxy group, an ester group,
the brace indicating where the group A is bound to the chitosan radical either directly or *via* an organic spacer group.

13. Compound according to claim 10, wherein the groups A consist of aromatic groups comprising several aromatic rings, said aromatic rings being fused, at least one of said rings comprising at least one imine group and at least one of said rings comprising at least one other group different from an imine group and comprising at least one atom bearing a lone pair.

14. Compound according to claim 13, wherein the groups A has the following formula (VII): 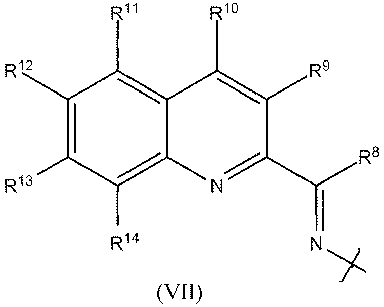 wherein:
- R⁸ represents a hydrogen atom or an alkyl group;
- R¹⁴ represents a group comprising an electron withdrawing oxygen atom and with a donor mesomeric effect;
- R⁹ to R¹³ represent, independently of each other, a hydrogen atom, an alkyl group, an alkoxy group, an ester group,
the brace indicating where the group A is bound to the chitosan radical either directly or *via* an organic spacer group.

15. Paper comprising at least one compound belonging to the chitosan family as defined according to any one of claims 10 to 14.
